# EUROPEAN PATENT SPECIFICATION

(11) **EP 4 005 608 B1**
(45) Date of publication and mention of the grant of the patent: **10.04.2024**
(21) Application number: 19765526.9
(22) Date of filing: 24.07.2019
(51) Int. Cl.: A61L 26/00, A61K 8/73, A61K 31/01, A61K 8/31, A61Q 19/00

(54) **NEW ANTIOXIDANT COMPOSITION FOR WOUND HEALING**
NEUE ANTIOXIDANTISCHE ZUSAMMENSETZUNG ZUR WUNDHEILUNG
NOUVELLE COMPOSITION ANTIOXYDANTE POUR LA CICATRISATION DE BLESSURES

(43) Date of publication of application: 01.06.2022
(73) Proprietor: Histocell, S.L., 48160 Derio / Bizkaia (ES)
(72) Inventor: HERRERO DE MIGUEL, Jone, 48600 Sopela (ES); CASTRO FEO, María Begoña, 48940 Leioa/Bizkaia (ES); BASTIDA CORCUERA, Felix Daniel, 20006 Donostia, Gipuzkoa (ES)
(74) Representative: Carlos Hernando, Borja
(86) International application number: PCT/ES2019/070520
(87) International publication number: WO 2021/014032

(56) References cited:
- EP-A1- 2 583 682
- WO-A2-2005/087208
- US-A1- 2010 330 135

## Description

### TECHNICAL FIELD OF THE INVENTION

The present invention is comprised in the cosmetic and/or therapeutic industry and specifically relates to a new composition for topical use with antioxidant and hydrating activity which promotes wound healing and is useful in the therapeutic and/or cosmetic treatment of skin lesions.

The novel composition object of the present invention incorporates certain agents and ingredients, among which are galactomannans and carotenoids, which surprisingly improve the healing and curing activity in a broad spectrum of wounds occurring with reactive oxygen species.

### BACKGROUND OF THE INVENTION

A wound or lesion in the skin is defined as the rupture of the mucosal cutaneous integrity, which can be caused intentionally, by trauma or by ischemia. Therefore, the wound is considered a deformity or interruption, which may affect from the epidermis to deeper structures.

The healing process is a local phenomenon in which elements common to several areas of the body participate, and it is easy to imagine that environmental and physiological factors have an enormous impact on healing progression and can affect the quality of the scar, over the time of healing and in the presence or absence of complications.

In this aspect, treating wounds is a challenge, primarily wounds considered to be chronic because they do not go through an orderly and timely process to promote anatomical and functional integrity, and they last for many months or even years, presenting frequent recurrences.

Given that these lesions are chronic and present a possibility of recurrence in a longer or shorter period of time, they may lead to psychosocial repercussions in patients to the extent that they may bring about lifestyle changes, prolong the time away from the family home, cause a change in how one sees oneself, which affects each individual differently, to a lesser or greater intensity, in addition to being a limitation for performing daily activities.

Therefore, the use of topical products to aid in the tissue repair process is of enormous value, since it is by means of the topical therapy that the ideal conditions for healing can be achieved.

It must be taken into account that the topical treatment of a wound indicates not only implanting the product in the bed of a lesion, but also evaluating the wound with regard to its state of healing, the tissue present in the bed, exudate, etc.

There is a wide range of products on the market for that purpose, there is known to be a range of about 2,000 products that are intended for treating wounds, from the simplest to the most complex covers that actively interfere in the various phases of the healing process in the various types of wounds.

Taking into account this great diversity of topical products with different indications and mechanisms of action, they are classified as, according to the characteristics of the wound, in epithelialising, absorbent, debriding, antibiotics and antiseptic products. Many of these products have a high cost, and others require the intervention of a professional to be suitably applied.

The healing process is a complex and fragile process; therefore, it is susceptible to being interrupted and failing, which leads to the formation of chronic wounds that do not heal or that acquire a state of pathological healing.

In general, the wound healing process requires suitable management of inflammation and the maintenance of a suitable environment (moisture, temperature, cleanliness), to maintain the stimulation of cell viability and the proliferation capacity thereof.

During the first phase of the healing process (inflammatory phase), the wound is exposed to reactive oxygen species, such as superoxide (O₂⁻), hydrogen peroxide (H₂O₂), as well as hydroxyl radicals (HO₂) and singlet oxygen (¹O₂).

These reactive oxygen species appear as a defence barrier against bacteria and as signalling molecules of the healing process. However, when they are not suitably regulated and an excess is produced, as occurs in the case of chronic wounds, these species cause serious damage in the wound and significantly slow down the healing process.

Antioxidants are well known in the state of the art as inhibitors of the damage caused by reactive oxygen species.

The state of the art discloses a number of compositions containing antioxidants for treating and curing wounds.

Patent US 7094431 describes a composition comprising zinc oxide, a calcium channel blocker, soluble vitamins combined with antibacterial and fungicidal agents.

Patent US 6046160 describes a composition comprising a D-glucose polysaccharide obtained by hydrolysis of starch, ascorbic acid, type I collagen, alpha tocopherol acetate.

Patent US 5874479 describes a composition for curing wounds comprising pyruvate, an antioxidant and a mixture of saturated and unsaturated fatty acids.

However, although the aforementioned inventions are reported as useful in inhibiting the production of reactive oxygen species during the wound curing process, none of them is shown to be completely satisfactory.

Lastly, patent EP2583682 describes an antioxidant composition comprising a combination of galactomannan and acetylcysteine for use in the treatment of wounds. The invention described in this patent represents the state of the art closest to the present invention from the composition viewpoint. As will be developed throughout the present specification, with respect to the object of the mentioned patent, the present invention presents a better and unexpected functionality entailing significant application advantages. Patent EP 2 583 682 A1 discloses an antioxidant composition comprising galactomannan and curcumin (turmeric), wherein the antioxidant effect is achieved by a synergy between the galactomannan and N-acetyl cysteine.

The present invention provides a composition with a substantially improved activity for treating and curing open wounds and burns, having a baseline antioxidant capacity that is superior to compositions of the state of the art. This new property of the composition translates into greater antioxidant effectiveness at a lower concentration of active ingredients.

### BRIEF DESCRIPTION OF THE INVENTION

The present invention relates to a new composition with antioxidant activity comprising a carotenoid combined with a galactomannan, and the use thereof to promote, accelerate and/or favour the healing of open wounds and cutaneous lesions in mammals, of any type, including burns, photoaging, radiation injuries, and acute and chronic wounds, and to prevent the formation of lesions in intact skin in mammals.

The authors of the present invention have discovered that the combination of the galactomannan locust bean gum and the carotenoid lycopene stimulates the cellular proliferation of fibroblasts and keratinocytes during the healing process as well as the closure of open wounds in a surprising manner compared to other antioxidant compositions of the state of the art.

The surprising curative effect of the composition of the present invention is due to a higher baseline antioxidant capacity, that is, the presence in the composition of certain hydrophilic and lipid antioxidants, specifically locust bean gum and lycopene combination which substantially increases the spectrum of the antioxidant capacity of the combination against reactive oxygen substances, in particular, against singlet oxygen, thereby preventing the drawbacks of previous treatments such as the loss of antioxidant activity and the inability to efficiently neutralise some reactive oxygen species, such as singlet oxygen. This higher baseline antioxidant capacity necessarily translates into greater effectiveness of the composition of the present invention, a lower concentration of active ingredients, and/or for a longer period of time.

Thus, the inventors have concluded that the selection of lycopene combined with locust bean gum, allows obtaining surprisingly satisfactory results in relation to other antioxidant compositions of the state of the art with respect to the stimulation of the cellular proliferation of fibroblasts and keratinocytes during the healing process and closure of open wounds. The present invention relates to an antioxidant composition comprising, with respect to the total weight of the composition, 0.1 - 5 % locust bean gum, 0.0001 - 1 % lycopene and 0.0001 - 1 % turmeric.

This is why one aspect of the present invention relates to the above disclosed antioxidant composition for use in the therapeutic treatment of skin lesions in mammals.

Also an object of the present invention is a composition as defined in any of the preceding paragraphs, wherein the composition may contain one or more additional carotenoids selected from the group comprising: alpha-carotene, beta-carotene canthaxanthin, cryptoxanthin, astaxanthin, capsanthin, zeaxanthin, lutein, apocarotenal violaxanthin.

Also an object of the present invention is a composition as defined in any of the preceding paragraphs, additionally comprising other active ingredients selected from the group comprising: micronutrients, vitamins, minerals, trace elements antibiotics and natural plant extracts.

Also an object of the present invention is a composition as defined in any of the preceding paragraphs, wherein the therapeutic treatment of the skin comprises improving the healing and curing process in wounds occurring with the presence of reactive oxygen species, including the healing of open wounds and cutaneous lesions in mammals, of any type, including burns, photoaging, radiation injuries, and acute and chronic wounds, and to prevent the formation of lesions in intact skin in mammals.

Also an object of the present invention is a composition as defined in any of the preceding paragraphs, wherein the treatment includes therapeutic and/or healing treatment for acute trauma and surgical wounds, cuts, abrasions, lacerations, sores, burns, scalds, skin irritation, fistulas, surgical dehiscences, radiation injuries, venous ulcers, arterial ulcers, pressure ulcers, diabetic foot ulcers, mixed aetiology ulcers, inflammatory skin lesions, skin cancer, and chronic or necrotic wounds.

Also an object of the present invention is a composition as defined in any of the preceding paragraphs, wherein the therapeutic treatment of the skin comprises improving the healing and curing process in wounds occurring with reactive oxygen species, including the healing of open wounds and cutaneous lesions in mammals, of any type, including burns, photoaging, radiation injuries, and acute and chronic wounds, and to prevent the formation of lesions in intact skin in mammals.

Also an object of the present invention is a composition as defined in any of the preceding paragraphs, wherein the treatment includes therapeutic and/or healing treatment for acute trauma and surgical wounds, cuts, abrasions, lacerations, sores, burns, scalds, skin irritation, fistulas, surgical dehiscences, radiation injuries, venous ulcers, arterial ulcers, pressure ulcers, diabetic foot ulcers, mixed aetiology ulcers, inflammatory skin lesions, skin cancer, and chronic or necrotic wounds.

Also an object of the present invention is a composition as defined in any of the preceding paragraphs formulated for topical use selected from the group comprising: cream, lotion, micro-emulsion, gel, hydrogel, ointment, liposomes, mists, powders and aqueous solutions or suspensions.

Also an object of the present invention is a composition as defined in any of the preceding paragraphs in the form of a hydrogel having a viscosity of 25000 to 70000 mPa·s (RV 05 5 rpm).

### DESCRIPTION OF THE FIGURES

Figure 1: Graph showing the results of the "wound closure or healing" assay described in Example 2 with respect to the cellular proliferation (% of area covered by fibroblasts) of two compositions containing the same concentration of galactomannan (0.2 % LBG) and a different concentration of N-acetylcysteine (NAC) or 0.001 % lycopene.

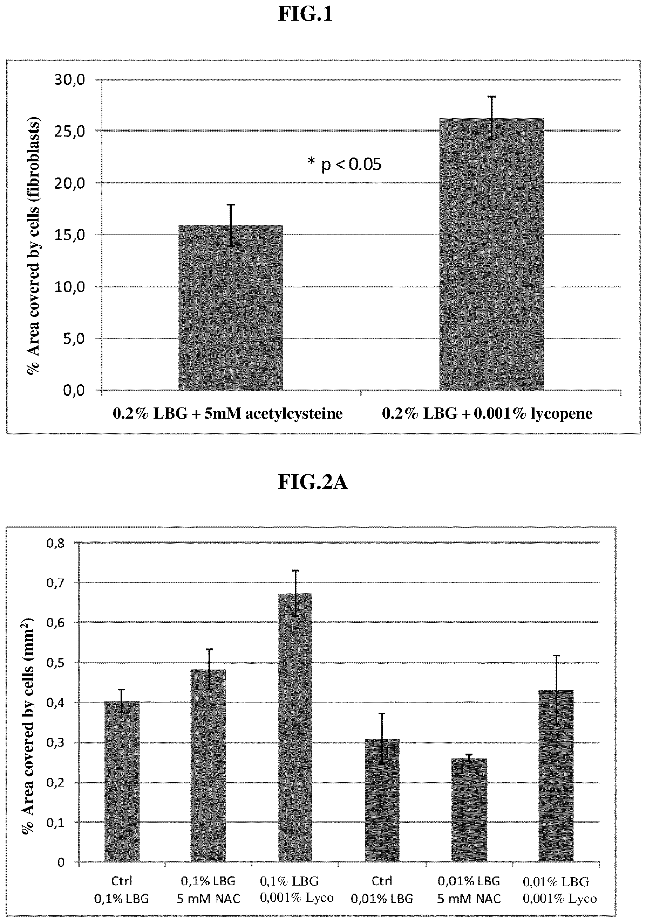

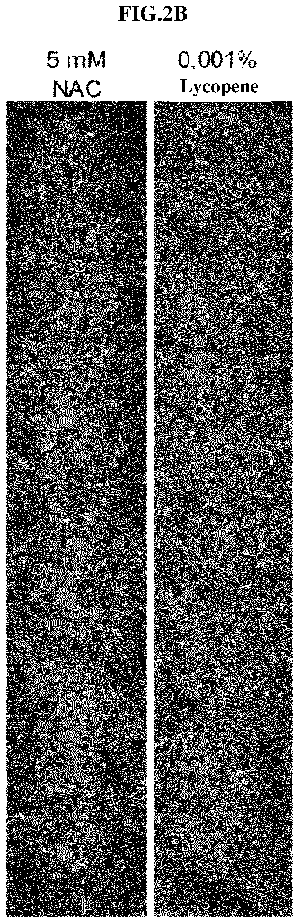

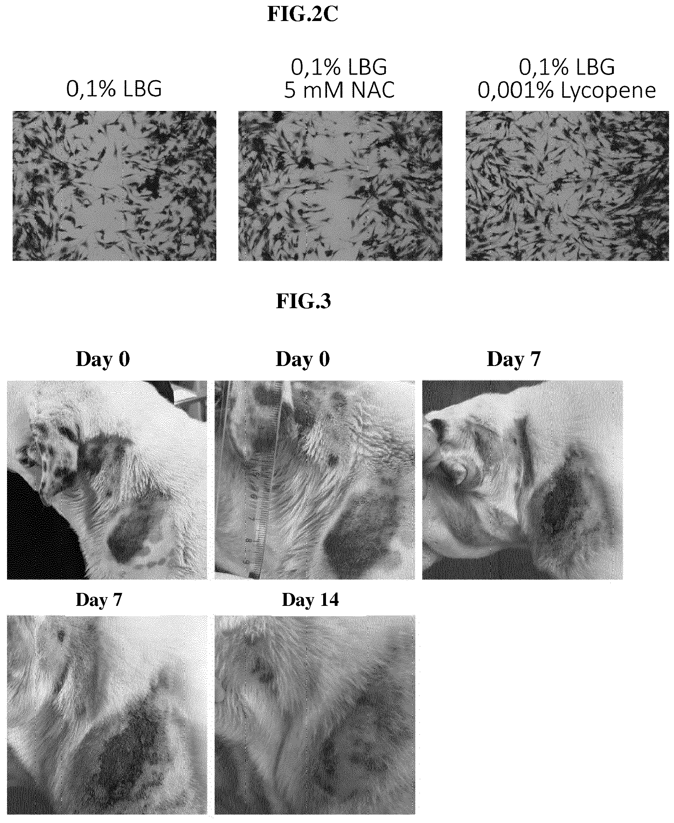

Figure 2 (2A-2C): Figure 2A relates to a graph showing the results of the "wound closure or healing" assay described in Example 2 with respect to the cellular proliferation of different compositions containing the same concentration of NAC and lycopene, (5 mM and 0.001 %, respectively) and a different amount of locust bean gum LBG, (0.1 % and 0.01 %). Figures 2B and 2C are images obtained by inverted optical microscopy relating to the assay of Example 2 showing the migration of fibroblasts in the *in vitro* wound model. Figure 2B shows the higher capacity of migration of the fibroblasts in the presence of 0.001 % lycopene with respect to the 5 mM NAC and Figure 2C shows the higher capacity of migration of the fibroblasts in the presence of 0.1 %LBG+0.001 % lycopene with respect to the 0.1 %LBG+5 mM NAC and 0.1 %LBG conditions.
Figure 3: Effect of treatment with the composition of the present invention in a clinical case: acute moist dermatitis in a dog.
Figure 4: Effect of treatment with the composition of the present invention in a clinical case: Trauma in the penis of a dog.
Figure 5: Graph of the results of the ORAC assay referred to in Example 4, which shows the antioxidant capacity over time of a formulation according to the present invention and a formulation in which lycopene is substituted with N-acetylcysteine.

### DETAILED DESCRIPTION OF THE INVENTION

The object of the present invention is a new composition with antioxidant activity comprising a carotenoid combined with a galactomannan and the use thereof to promote, accelerate and/or favour the healing of open cutaneous wounds and /or burns in mammals. In other words, the present invention relates to a composition for topical use with antioxidant and hydrating activity which promotes wound healing and is useful in the therapeutic treatment of skin lesions in mammals, surprisingly improving the healing and curing process in wounds occurring with a broad spectrum of reactive oxygen species, including the healing of open wounds and cutaneous lesions in mammals, of any type, including burns, photoaging, radiation injuries, and acute and chronic wounds, and to prevent the formation of lesions in intact skin in mammals.

As discussed in preceding sections, during the first phase of the healing process the wound is exposed to reactive oxygen species, such as superoxide (O₂⁻), hydrogen peroxide (H₂O₂), as well as hydroxyl radicals (HO₂) and singlet oxygen (¹O₂). Therefore, any composition intended for wound healing and/or curing must, among others, neutralise the excess of said reactive oxygen species to minimise the oxidative damage caused by same.

The surprising curative effect of the composition of the present invention is due to a higher baseline antioxidant capacity, that is, the presence in the composition of certain hydrophilic and lipid antioxidants, specifically the combination locust bean gum and the carotenoid lycopene substantially increases the spectrum of the antioxidant capacity of the combination against reactive oxygen substances, in particular, against singlet oxygen, thereby preventing the drawbacks of previous treatments such as the loss of antioxidant activity and the inability to efficiently neutralise the significant singlet oxygen.

Carotenoids are organic pigments occurring naturally in plants and other photosynthetic organisms such as algae, some classes of fungi and bacteria.

Carotenoids belong to the group of terpenoids, which are a series of compounds that come from mevalonic acid (Acetyl CoA derivative) and are isoprene derivatives, a hydrocarbon consisting of five carbon atoms. Specifically, carotenoids have an isoprenoid structure, are tetraterpenes and are made up of forty carbon atoms. Those atoms form conjugated chains that can end in substituted and unsaturated carbon rings at each of their ends.

According to their chemical structure carotenoids can be classified as carotenes and xanthophylls. Carotenes are carotenoids that do not contain oxygen and xanthophylls are carotene derivatives that contain oxygen. The compositions of the present invention contain lycopene, and optionally, one or more carotenoids selected from the group comprising: astaxanthin, cryptoxanthin, canthaxanthin, capsanthin, lutein, violaxanthin, beta-carotene and alpha-carotene. In a particular embodiment, lycopene is in its pure extract or hydrophilic dispersion form. Carotenoids are obtained through techniques that are well known to one skilled in the art. The most commonly used methods are plant material extraction processes, such as extraction by solvents, supercritical fluids, etc., or by means of microbiological methods or chemical synthesis.

Lycopene is found in tomatoes and other red fruits and vegetables, such as red carrots, red pepper, watermelon, and papayas. In particular, lycopene can be extracted from the whole fruit, pulp, skins or seeds of the tomato by extraction with suitable solvents.

A method for obtaining lycopene can be carried out using whole fruits of *Lycopersicum esculentum* and/or similar species or parts thereof, coming from food industry process byproducts. In this case, the partially dehydrated fresh material is subjected to extraction with aromatic or aliphatic hydrocarbons or with water-immiscible solvents, in the presence of phospholipids, such as stabilising agents and surfactants. The extracts are concentrated until forming an oil or fractionated until obtaining the required concentration of lycopene. For purposes of the present invention, natural or synthetic extracts, dispersions, etc. with a concentration of lycopene between 0.0001 and 1.0 %, preferably 0.001 %, can be used. Lycopene products available on the market are: Redivivo^{®} Lycopene marketed by DMS Nutritional products AG; TomatoRed^{®} 10 % CWD by Lycored; LycoVit^{®} 10 CWD/S or LycoVit^{®} 10 % DC by BASF etc.

Lycopene can also be obtained from biosynthetic natural sources, such as submerged cultures of fungi such as, *Blakeslea, Choanephora* or *Phycomyces.* In this case, the method consists of the following steps: Direct treatment with alcohol on the source natural of biosynthesis (fermentation broth) and separation of a purified wet biomass. Conditioning of the purified biomass is then produced by means of drying plus the breaking up or rupture of the biomass, solid-liquid extraction of lycopene with an organic solvent, concentration of the enriched extract, precipitation/crystallisation by adding alcohol, filtration, and finally, drying. In a particular embodiment of the present invention, the composition may contain mixtures of carotenoids selected from the group comprising: astaxanthin, lutein, violaxanthin, beta-carotene, alpha-carotene and lycopene, where the composition contains at least the carotenoid lycopene. In another particular embodiment of the present invention, the composition only contains the carotenoid lycopene. In a particular embodiment, the carotenoid forming part of the composition of the present invention is beta-carotene and lycopene, in its pure extract or hydrophilic dispersion form. Lycopene is present in the composition in a proportion of between 0.0001 and 1.0 % by weight with respect to the total weight of the composition. More preferably, the compositions according to the present invention comprise lycopene in the range of 0.0001 - 0.01 % by weight with respect to the total weight of the composition. In the present invention, the composition comprises lycopene in a proportion of 0.0001 to 1.0 % by weight with respect to the weight of the final composition.

Galactomannans are biopolymers of the polysaccharide type formed by a mannose backbone with branches formed by galactose units. More specifically, the backbone is formed by β-D-mannopyranose units attached by (1→4) bonds, with α-D-galactopyranose branches attached by (1→6) bonds.

Galactomannans that are useful for purposes of the present invention comprise natural and/or modified galactomannan. According to the present invention, the galactomannan of the composition is locust bean seed gum.

Suitable modified polysaccharide gums include esters of natural or substituted polysaccharide gums, such as carboxymethyl esters, ethylene glycol esters and propylene glycol esters. An example of substituted polysaccharide gum is methyl cellulose. The composition of the present invention may contain a single gum or a mixture of several gums as defined in preceding sections.

The gums and particularly galactomannan gums are well-known materials. See, for example, Industrial Gums: Polysaccharides & Their Derivatives, Whistler R.L. and BeMiller J.N. (eds.), 3rd ed. Academic Press (1992) and Davidson R.L., Handbook of Water-Soluble Gums & Resins, McGraw-Hill, Inc., N.Y. (1980). Most gums are available on the market in several forms, usually in powder form, and are easy to use in foods and topical compositions. For example, locust bean seed gum in the form of powder available on the market is: Seedgum^{®} by LBG SICILIA SRL, Italy; different products marketed by ILCAR, S.r.l., Italy, etc.

There are various methods for obtaining locust bean gum: the alkaline chemical extraction method and the dry mechanical method.

In the alkaline chemical method, the selected and oven-dried fruits were immersed in a solution of 25 % NaOH for 5 days to extract the seeds. Subsequently, they were subjected to moist heat at a temperature of 80±2 °C using 0.75 % NaOH for 10 to 15 minutes, by means of stirring. They were washed with abundant water until observing detachment of the seeds and were left to soak for 24 hours, changing the water periodically. The endosperm was manually separated from the testa, tegument and cotyledon, taking it to an oven at 35±2 °C for 16 hours, and was milled in an electric mill and sieved through a No. 60 mesh measuring 250 microns to obtain the gum.

In the dry mechanical method, the selected and dried fruits were milled and sieved, separating the exocarp and mesocarp of the seed, which were oven-dried for 16 hours and then milled and sieved, separating the testa, tegument and cotyledon from the endosperm, which was milled in an electric mill until obtaining the gum which was sieved using a No. 60 mesh measuring 250 microns.

The composition of the present invention comprises locust bean gum in a proportion in a range of 0.1 - 5 % by weight with respect to the total weight of the composition, more preferably, 0.5 - 3 % by weight with respect to the total weight of the composition, lycopene in a proportion by weight of between 0.0001 and 1.0 % by weight with respect to the total weight of the composition and tumeric in a proportion of between 0.0001-1% with respect to the total weight of the composition.

Optionally, the compositions of the present invention may contain in addition to the lycopene and locust bean gum and tumeric, other active ingredients selected from the group comprising: turmeric, other antioxidant agents, such as ascorbic acid, tocopherols, alpha-lipoic acid, anthocyanins, gallic acid, epigallocatechin gallate (EGCG), ferulic acid, coenzyme Q10, squalene, flavonoids, resveratrol, polyethylene glycol, selenium, cysteine, micronutrients, such as vitamins, minerals and trace elements, antibiotics and natural plant extracts with an activity that heals or is beneficial for the skin (aloe vera, *Centella asiatica,* Hamamelis, *Plantgo lanceolata,* Lavandula, *Echinacea angustifolia, Symphytum officinale, Chamaemelum nobile, Calendula officinalis* and *Helichrysum italicum*)*.*

Turmeric is an herbaceous plant of the ginger family native to southwest India. The extract of this plant can be used in two ways: as turmeric (raw extract), and as curcumin (purified or refined state). The methods of obtaining such ingredient are well known to one skilled in the art. Furthermore, there are a number of commercial turmeric products that can be used for the purposes of the present invention, such as for example those marketed by the following companies: NovaSol^{®} by AQUANOVA AG, Germany; as well as other products marketed by Undersun Biomedtech Corp, CA, USA; Xian Geekee Biotech Co., Ltd., China; PROQUIMAC PFC, S.A., Spain; Himachal Pharmaceuticals, India; Sancolor, S.A., in Spain.

In those embodiments as described in detail in preceding paragraphs in which turmeric dissolved in ethanol is incorporated in the composition comprising a locust bean gum and lycopene, the proportion by weight of turmeric in the composition of the invention is between 0.0001 and 1.0 % with respect to the total weight of the composition.

In the present invention, the composition comprises locust bean gum in a proportion by weight of between 0.1 and 5.0 % by weight with respect to the total weight of the composition, lycopene in a proportion by weight of between 0.0001 and 1.0 % by weight with respect to the total weight of the composition and turmeric in a proportion by weight of between 0.0001 and 1.0 % by weight with respect to the total weight of the composition.

With respect to the vitamins which may form part of the formulation in the composition of the present invention, the following are contemplated: water-soluble vitamins, such as B-complex vitamins (thiamine, riboflavin, niacin, pyridoxine, folate, vitamin B12, biotin, pantothenic acid) and vitamin C; and liposoluble vitamins such as vitamin A, vitamin D, vitamin E and vitamin K, as well as their precursors, analogues and derivatives. A preferred embodiment of the present invention contemplates the incorporation of alpha-tocopherol (vitamin E) and ascorbic acid (vitamin C) in the composition. These ingredients are available on the market, such as through the following companies for example: Koninklijke DSM N.V., Netherlands; NHU EUROPE GmbH, Germany; PMC Isochem, France; Alpha Environmental, USA; Spectrum Chemical Mfg. Corp, USA.

In those embodiments as described in detail in preceding paragraphs in which vitamins are incorporated in the composition comprising a carotenoid and galactomannan, the proportion by weight of the vitamins, their precursors, analogues and derivatives in the composition of the invention is between 0.0001 and 5.0 % with respect to the total weight of the composition.

Likewise, in those embodiments as described in detail in preceding paragraphs in which vitamins are incorporated in the composition comprising locust bean gum, lycopene and turmeric, the proportion by weight of the vitamins, their precursors, analogues and derivatives in the composition of the invention is between 0.0001 and 5.0 % with respect to the total weight of the composition.

Also in those embodiments as described in detail in preceding paragraphs in which vitamins are incorporated in the composition comprising lycopene, locust bean gum and turmeric, the proportion by weight of the vitamins, their precursors, analogues and derivatives in the composition of the invention is between 0.0001 and 5.0 % with respect to the total weight of the composition.

The active ingredients forming part of the compositions of the present invention are administered to the affected area of the skin in a pharmaceutically acceptable topical excipient. For purposes of the present invention, a pharmaceutically acceptable topical excipient is any pharmaceutically acceptable formulation that can be applied to the surface of the skin for topical, dermal, intradermal or transdermal administration of the compositions described in the present invention.

The topical formulations of the invention are prepared by mixing the ingredients and/or mixtures of ingredients of the compositions as described above with a topical excipient according to the methods that are well known in the art, for example, the methods provided by conventional reference texts, such as REMINGTON: THE SCIENCE AND PRACTICE OF PHARMACY (Alfonso R. Gennaro ed. 19th ed. 1995); Ghosh, T. K.; et al.

The topical excipients useful for the topical administration of the formulations and compositions of the present invention described above can be any excipient known in the art for topical administration, for example, creams or lotions; micro-emulsions; gels; hydrogels, ointments; liposomes; powders and aqueous solutions or suspensions.

In a preferred embodiment, the topical excipient used to administer a compound described above is an emulsion, gel, hydrogel or ointment. Emulsions such as creams and lotions are topical formulations suitable for use according to the invention. Emulsions such as creams and lotions which can be used as topical excipients and their preparation are described in REMINGTON: THE SCIENCE AND PRACTICE OF PHARMACY 282-291 (Alfonso R. Gennaro ed. 19th ed. 1995).

In another embodiment, the topical excipient used to administer the formulation or composition described above is a gel, for example, a two-phase gel or a single-phase gel. The gels and hydrogels suitable for use in the invention are described in REMINGTON: THE SCIENCE AND PRACTICE OF PHARMACY 15171518 (Alfonso R. Gennaro ed. 19th ed. 1995).

In another embodiment, the topical excipient used to administer the formulation or composition described above is a mixture for administration in the form of a spray with atomisers or containers with an airless system. With respect to the latter, it should be pointed out that "the airless system" has an appearance similar to aerosols but differ from conventional containers in relation to their metering system. They work with a continuous vacuum system, so the use of propellants or preservatives is not required. The product containing it does not come into contact with air nor is it handled directly by hand, such that it is free of bacterial contamination. As it is consumed, the internal piston pushes the content upwards, therefore product is not wasted. One skilled in the art knows the techniques as well as the excipients necessary for suitably formulating the composition of the present invention in the discussed administration formats.

In general, polymer thickeners (gelling agents) that can be used for purposes of the present invention include those known to one skilled in the art, such as hydrophilic and hydroalcoholic gelling agents commonly used in the cosmetic and pharmaceutical industries. Preferably, the hydrophilic or hydroalcoholic gelling agent comprises "CARBOPOL^{®}" (B.F. Goodrich, Cleveland, Ohio), "HYPAN^{®}" (Kingston Technologies, Dayton, N.J.), "NATROSOL^{®}" (Aqualon, Wilmington, Del.), "KLUCEL^{®} "(Aqualon, Wilmington, DE) or "STABILEZE^{®}" (ISP Technologies, Wayne, N.J.). Preferably, the gelling agent comprises between about 0.05 % and about 4 % by weight of the composition. More particularly, the preferred percent range by weight of the composition for "Carbopol^{®}" is comprised between about 0.2 % and about 0.6 %.

"Carbopol^{®}" is one of a number of cross-linked polymers of acrylic acid referred to with the general name carbomer. These polymers are dissolved in water and form a transparent or slightly turbid gel in neutralisation with a caustic material such as sodium hydroxide, potassium hydroxide, triethanolamine, or other amine bases.

In another preferred embodiment, the topical excipient used to administer a compound described above is an ointment. Ointments suitable for use in the invention are well known in the art and are described in REMINGTON: THE SCIENCE AND PRACTICE OF PHARMACY 1585-1591 (Alfonso R. Gennaro ed. 19th ed. 1995).

In another embodiment, the topical excipient used in the topical formulations of the invention is an aqueous solution or suspension, preferably, an aqueous solution. Aqueous topical formulations suitable for use in the invention are described in REMINGTON: THE SCIENCE AND PRACTICE OF PHARMACY 1563-1576 (Alfonso R. Gennaro ed. 19th ed. 1995).

The pH of the topical formulations of the invention are preferably in the range from about 3 to about 8, more preferably, from about 5.5 to about 6.5. To stabilise the pH, an effective amount of a buffer is preferably included. In one embodiment, the buffer agent is present in the topical formulation in an amount from about 0.05 % to about 1 % by weight of the formulation. To adjust the pH, acids or bases can be used as needed, such as, for example, hydrochloric acid, citric acid, lactic acid, acetic acid, sodium hydroxide, sodium bicarbonate, sodium carbonate, dibasic sodium phosphate, sodium borate, etc.

Preferably, the topical formulations in the form of gel or hydrogel of the invention have a viscosity comprised in the range of 25 to 70 Pa·s (around 25000 to 70000 Pa s).

The topical formulations of the invention may comprise pharmaceutically acceptable excipients such as those listed in REMINGTON: THE SCIENCE AND PRACTICE OF PHARMACY (Alfonso R. Gennaro ed. 19th ed. 1995), including but not limited to, protectors, adsorbents, lenitives, emollients, preservatives, humectants, buffering agents, solvent agents, skin penetrating agents, and surfactants.

The compositions of the present invention may contain additional antioxidants, such as ascorbic acid and its esters, sodium bisulfite, butylated hydroxytoluene, butylated hydroxyanisole, tocopherols, and chelating agents such as EDTA and citric acid.

Suitable humectants include, but are not limited to, glycerin, sorbitol, polyethylene glycols, urea and propylene glycol.

Buffering agents suitable for use in the invention include, but are not limited to, acetate buffers, citrate buffers, phosphate buffers, lactic acid buffers and borate buffers.

Suitable solvent agents include, but are not limited to, quaternary ammonium chlorides, cyclodextrins, benzyl benzoate, lecithin and polysorbates.

Suitable skin-penetrating agents include, but are not limited to, ethyl alcohol, isopropyl alcohol, octyl phenyl polyethylene glycol, oleic acid, polyethylene glycol 400, propylene glycol, N-decylmethyl sulfoxide, fatty acid esters (for example, isopropyl myristate, methyl laurate, glycerol monooleate, and propylene glycol monooleate), and N-methylpyrrolidone.

In a preferred embodiment of the present invention, the composition is presented in gel form and comprises the following ingredients and their proportion by weight with respect to the total weight of the composition:
0.1 % - 1.0 % Carbomer
0.05 % - 0.5 % EDTA
1.0 % - 20.0 % Glycerol
0.05 % - 5.0 % Locust bean gum
0.0001 % - 0.01 % Turmeric
0.0001 % - 1.0 % Lycopene
0.1 % - 10.0 % Ascorbic acid
0.0001 % - 2.5 % Alpha tocopherol
q.s. H₂O

In another preferred embodiment of the present invention, the composition is presented in gel form and comprises the following ingredients and their proportion by weight with respect to the total weight of the composition:
0.1 % - 1.0 % Carbomer
0.05 % - 0.5 % EDTA
1.0 % - 20.0 % Glycerol
0.05 % - 5.0 % Locust bean gum
0.0001 % - 1.0 % Lycopene
0.1 % - 10.0 % Ascorbic acid
0.0001 % - 2.5 % Alpha tocopherol
q.s. H₂O

In another composition not part of the invention, the composition is presented in gel form and comprises the following ingredients and their proportion by weight with respect to the total weight of the composition:
0.1 % - 1.0 % Carbomer
0.05 % - 0.5 % EDTA
1.0 % - 20.0 % Glycerol
0.05 % - 5.0 % Guar gum
0.0001 % - 0.01 % Turmeric
0.0001 % - 1.0 % Beta-carotene
0.1 % - 10.0 % Ascorbic acid
0.00001 % - 2.5 % Alpha tocopherol
q.s. H₂O

In another composition not part of the invention, the composition is presented in gel form and comprises the following ingredients and their proportion by weight with respect to the total weight of the composition:
0.1 % - 1.0 % Carbomer
0.05 % - 0.5 % EDTA
1.0 % - 20.0 % Glycerol
0.05 % - 5.0 % Guar gum
0.0001 % - 1.0 % Beta-carotene
0.1 % - 10.0 % Ascorbic acid
0.00001 % - 2.5 % Alpha tocopherol
q.s. H₂O

The topical formulations of the invention are prepared by mixing the ingredients of the compositions as described above with the pharmaceutically and/or cosmetically acceptable topical excipients and additives as follows: The carbomer is hydrated in half of the final volume of water under gentle continuous stirring (mixture A). The locust bean gum is mixed with the glycerol and 40 % of the final water is added by stirring (mixture B). Once the carbomer is correctly hydrated, the different components are added sequentially to mixture A under stirring. Among these components the following are included: EDTA, turmeric, ascorbic acid, alpha-tocopherol, lycopene, other carotenoids, etc. Both mixtures (A and B) are combined, and once good product homogeneity has been achieved, the carbomer is activated and the pH is adjusted with sodium hydroxide or another pH corrector compatible with the carbomer until achieving the final pH of the sample, and the rest of the necessary water is added.

The compositions of the present invention are intended for the therapeutic use on the skin. Said cosmetic and/or therapeutic application will comprise any beneficial effect for the skin, the purpose of which is to improve or treat, including even cure, any skin condition or damage to the skin which requires promoting, accelerating and/or favouring the repair, hydration and healing of open cutaneous wounds and /or burns in mammals, regardless of the origin of the lesion, level of severity and/ or affected surface. The damage in the skin can range from a superficial irritation, lacerations, to deep damage affecting the dermis, epidermis and other deeper tissues such as muscle, fascias, conjunctive tissue, which even leaves bone and tendons and other types of subcutaneous structures exposed.

Specifically, application of the formulations of the present invention for the therapeutic and/or healing treatment for acute trauma and surgical wounds, burns, scalds, fistulas, surgical dehiscences, venous ulcers, arterial ulcers, pressure ulcers, diabetic foot ulcers, mixed aetiology ulcers, inflammatory skin lesions, moisture lesions, irritations, lacerations, avulsions, incisions, punctures, bites, skin cancer, and chronic or necrotic wounds.

Furthermore, the application of the formulations of the present invention for the treatment of skin damage related to age and caused by the presence of free oxygen radicals is contemplated, among others, aging of the skin due to intrinsic and/or extrinsic causes, such as sun, poor nutrition, environmental pollution, stress, tobacco, drugs, alcohol, dry skin, inflammatory diseases, dermatitis, loss of elasticity and collagen content in the skin.

To treat the skin disorders described in detail in the preceding paragraph according to the present invention, the compositions are applied topically on the affected area. For example, washing the area of application first, if necessary, with a soapy solution or an antiseptic followed by abundant physiological saline solution, Ringer's lactate or another suitable solution. After carefully drying the affected area with sterile gauze, the product would be applied on the damaged area, covering it with a layer of the product.

Generally, the amount of composition applied to the skin according to the invention varies depending on the pharmaceutical or cosmetic form of the product and the characteristics of the skin lesion, but, for example, for a hydrogel it could range between a thin layer of 0.1 cm or a thicker layer of up to 5 cm or more. In the case of a cavitated wound, the hydrogel must completely cover the cavity. Depending on the type of lesion and on whether or not the affected skin area is covered, the application regimen may vary between 2 to 6 times a day, or every 2 or 3 days during a period of time generally ranging between 3 and 60 days until the skin lesion improves or is cured, although in some cases the application can be prolonged for several months.

The therapeutic effect produced by the compositions of the present invention has surprising and substantial advantages over other similar treatments known in the state of the art. The state of the art closest to the invention is represented by European patent EP1206271B1, which describes an antioxidant composition for the cosmetic and/or therapeutic treatment of the skin comprising, among others, a galactomannan, such as locust bean gum, N-acetylcysteine and, optionally, turmeric.

The compositions of the present invention show a biological effect that is surprisingly superior to the compositions mentioned in EP1206271B1, based on an increase in antioxidant capacity entailing an increase of the healing activity of between 10 and 30 %, which represents advantageous consequences that can be applied to the treatment, among others, a decrease of the duration of treatment and a reduction of the time the patient suffers pain. Additionally, the compositions of the present invention show greater stability, which affects both the efficacy and the duration of their effect during the time of contact with the skin. Likewise, in relation to producing, storing and distributing the product, its greater stability provides it with competitive, economic and logistic advantages.

### EXAMPLES

### Example 1: Composition formulation

The product is mixed in two phases, adding the EDTA, turmeric, lycopene, ascorbic acid, tocopherol to a hydrated solution of the carbomer. The locust bean galactomannan is wetted in glycerol and then hydrated with 40 % of the final water.

Both phases are mixed and gelling of the carbomer is activated with a solution of sodium hydroxide until obtaining pH 6.5. Thus, the product obtained would consist of a composition in the form of a hydrogel with the following formulation:
0.3 % Carbomer
0.1 % EDTA
5.0 % Glycerol
3.0 % locust bean gum
0.16 % ethanol + 0.0001 % turmeric
0.0001 % lycopene
0.15 % ascorbic acid
0.02 % alpha tocopherol
NaOH Adjusted to 6.5
H₂O To complete weight, q.s.

Once the product is sterilised by moist heat, the initial viscosity is between 25000-35000 mPa·s (RV 05 5 rpm). Over time and due to the property inherent to non-Newtonian fluids prepared with locust bean galactomannan (thixotropy), the viscosity of the hydrogel increases until reaching viscosities of 70000 mPa·s (RV 05 5 rpm).

### Example 2: In vitro wound healing assay or scratch assay

The *in vitro* wound healing assay or scratch assay measures the capacity of a product to stimulate cellular proliferation (epithelial fibroblasts or keratinocytes) and close a gash without cells in a cellular monolayer in the culture tissue. For the gash to be homogeneous in all the wells, the cells are seeded in wells containing silicone blocks of the same size, which prevent adhesion of the cells in the area occupied by the block. Thus, epithelial fibroblasts were seeded in 24-well plates with the silicone blocks or barriers. After incubating for 12 hours, the barriers were removed and the cells were incubated for 24-72 hours with medium that contained the components separately or combined [different concentrations of locust bean galactomannan (LBG, 0.2, 0.1 and 0.01 %), lycopene (0.001 %)] and acetylcysteine (NAC, 5 mM). The entire surface of the well covered by the cells or the number of cells was estimated based on the images obtained of the wells with different treatments.

The results of the assay performed are shown in Figure 1, which illustrates the area of the surface of the wells that was covered with the fibroblasts that migrated to the area of the gash. It can be observed that wells treated with 0.2 % galactomannan and 0.001 % lycopene show a larger number of cells that migrated, such that they were able to cover a significantly larger area than if they were treated with 0.2 % galactomannan and 5 mM acetylcysteine. This *in vitro* result indicates that cells in the presence of 0.2 % locust bean gum and 0.001 % lycopene have a higher capacity to cover a wound *in vivo.*

Subsequently, it could be determined, as shown in Figure 2A to 2C, that the combination between the locust bean gum and lycopene produced the best results in the *in vitro* healing assay given that the area covered by cells was significantly greater under this condition with respect to the locust bean gum alone or the galactomannan+NAC combination (Figure 2A). Figures 2B and 2C show images of the healing model *in vitro,* where the area of the gash and its colonisation by fibroblasts can be observed. It can be observed how the 0.001 % lycopene induced *in vitro* healing better with respect to 5 mM NAC (Figure 2B) and that with the 0.1 % galactomannan+0.001 % lycopene combination better results were obtained results with regard to healing, with respect to 0.1 % locust bean gum and 0.1 % locust bean gum 5 mM NAC (Figure 2C).

### Example 3: Clinical trial in animals

A clinical field study was conducted (internal protocol PRO-12) with veterinarians specialising in treating skin wounds, to test the efficacy of the antioxidant composition of the present invention (formulation of Example 1) compared to a commercial hydrogel, (Vexoderm Gel^{®}), composition of which comprises galactomannan, N-acetylcysteine and turmeric. The summary of the qualitative results obtained are shown in detail below in Table 1.

**Table 1: Summary of the qualitative results of the assay comparing the clinical characteristics observed in animals treated with a formulation of EP1206271B1 (galactomannan + acetylcysteine + turmeric) and a gel formulation according to the present invention (galactomannan + lycopene + turmeric) (++++ excellent; +++ very good; ++ good; + suitable; - negative).**

| **Clinical parameter** | **Vexodenn Gel (acetylcysteine)** | **New hydrogel (example 1) (Lycopene)** | **Notes** |
|---|---|---|---|
| Ease of administration | +++ | +++ | The new hydrogel with lycopene is more fluid |
| Debriding/elimination of necrotic tissues/fibrin/sphacelus | ++ | +++ | |
| Cleanliness of the wound | ++ | +++ | |
| Granulation tissue | ++ | ++++ | Excellent granulation tissue with the new hydrogel which appears very rapidly. |
| Condition of the re-epithelialisation borders | ++ | ++++ | |
| Perilesional tissue | +++ | ++++ | |
| Closure of the wound | ++ | ++++ | The hydrogel with lycopene achieves closure of the wounds between 10-30 % faster than the hydrogel with acetylcysteine. |

The characteristics of two of the clinical cases in which the gel formulated according to the present invention was applied are shown below. The clinical cases were carried out by veterinarians with prior experience in using the hydrogel with a formulation indicated in EP1206271B1, such that a comparison between the two types of formulations could be performed.

### Case of a dog, English bulldog, acute moist dermatitis (hot spots)

An unneutered 4-year old English bulldog was taken to the clinic for pruritus and a foul-smelling lesion in the neck area. The area presented a rank smell, significant erythema, hair loss and somewhat bloody exudate. After closer inspection, two painful areas of moisture lesions inflamed with pus and exudate were detected. This type of lesion is typical of acute moist dermatitis (hot spot). The area was shaved and cleaned. After disinfecting the area, the composition (Example 1), i.e., hydrogel formulated with locust bean gum and lycopene, was applied in abundance. Systemic antibiotics and NSAIDs were prescribed and the owner was advised to: 1) wash the area every 12 hours; 2) for the two first days, also apply a topical corticosteroid cream to control pruritus; 3) followed by the galactomannan and lycopene hydrogel two hours after the cream. As shown in Figure 3, after 7 days, a significant improvement of the lesions was observed, and they were considered closed on day 14 after starting treatment with the hydrogel of the present invention.

### Case of trauma in the penis of a dog.

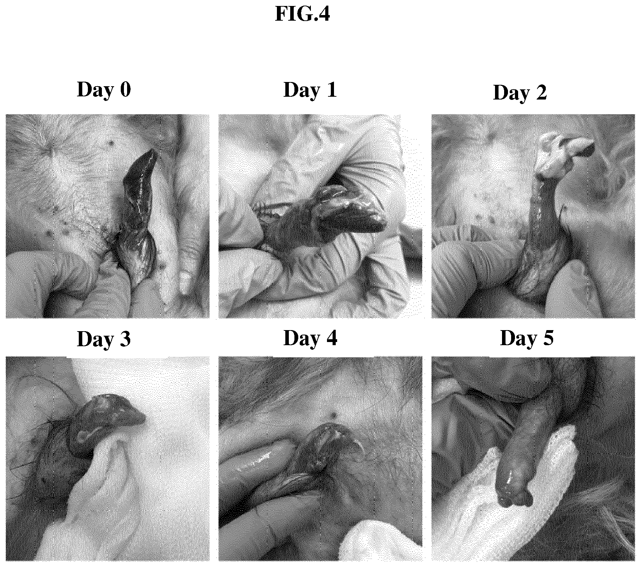

A 4-year old male canine patient with a large necrotic lesion in the penis probably due to trauma. During a rehabilitation session for a paraplegic lesion in the animal, for treatment, the area of the ulcer was cleaned with saline solution and sterile gauzes, the composition of the present invention (example 1), i.e., the hydrogel formulated with locust bean galactomannan and lycopene, was applied directly, and it was covered with a petrolatum-impregnated gauze dressing. Given that the dressing frequently became wet due to contact with urine, the veterinarian advised the owners to frequently wash the area, apply the hydrogel of the present invention and cover it. As can be seen in Figure 4, curing occurred rapidly, achieving complete elimination of the necrotic tissue on the third day and complete curing of the wound on day 6 after starting treatment, resulting in a recovered functional penis.

### Example 4: Stability assay on the antioxidant capacity of the formulation

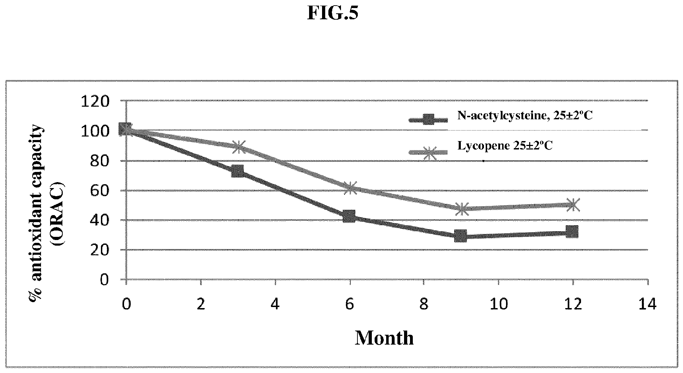

This study analysed the stability at 25±2 °C of identical formulations of the hydrogel, except for the inclusion of acetylcysteine (5 mM) or its substitution by the combination of lycopene (0.0001), vitamin C (0.15) and vitamin E (0.02). The analyses was performed in different tubes at the following times: 3, 6, 9 and 12 months. At these times, antioxidant capacity was determined by means of the ORAC assay and using Trolox^{®} (a water-soluble vitamin E derivative) to generate a standard curve. The data of each hydrogel was standardised at each of the analysis times to the initial value (time zero). The results demonstrate that the formulation of the present invention maintains better antioxidant capacity over time, i.e., the present formulation is more stable than the formulation with acetylcysteine as an antioxidant (Figure 5).

## Claims

1. An antioxidant composition comprising, with respect to the total weight of the composition, 0.1 - 5 % locust bean gum, 0.0001 - 1 % lycopene and 0.0001 - 1 % turmeric.

2. The antioxidant composition according to claim 1 for use in the therapeutic treatment of skin lesions in mammals.

3. The antioxidant composition for use according to claim 2, wherein the therapeutic treatment of the skin comprises improving the healing and curing process in wounds occurring with the presence of reactive oxygen species, including the healing of open wounds or and cutaneous lesions in mammals, of any type, including burns, photoaging, radiation injuries, and acute and chronic wounds, and preventing the formation of lesions in intact skin in mammals

4. The antioxidant composition for use according to any of claims 2 or 3, wherein the treatment includes therapeutic and/or healing treatment for acute trauma and surgical wounds, cuts, surgical dehiscences, abrasions, lacerations, moisture lesions, sores, burns, scalds, skin irritation, fistulas, venous ulcers, arterial ulcers, pressure ulcers, diabetic foot ulcers, mixed aetiology ulcers, inflammatory skin lesions, skin cancer, and chronic or complex wounds.

5. The antioxidant composition for use according to any of claims 2-4, wherein the use is topical use selected from the group comprising: cream, lotion, microemulsion gel, hydrogel, ointment, liposomes, powders and aqueous solutions or suspensions.

6. The antioxidant composition for the use according to claim 5 in the form of a hydrogel having a viscosity of 25000 to 70000 mPa·s (RV 05 5 rpm).

## Patentansprüche

1. Antioxidanszusammensetzung, die, bezogen auf das Gesamtgewicht der Zusammensetzung, 0,1-5 % Johannisbrotkernmehl, 0,0001 - 1 % Lycopin und 0,0001 - 1 % Kurkuma umfasst.

2. Antioxidanszusammensetzung nach Anspruch 1 zur Verwendung bei der therapeutischen Behandlung von Hautläsionen bei Säugern.

3. Antioxidanszusammensetzung zur Verwendung nach Anspruch 2, wobei die therapeutische Behandlung der Haut die Verbesserung des Heilungs- und Verheilungsprozesses bei Wunden umfasst, die in Anwesenheit reaktiver Sauerstoffspezies auftreten, einschließlich der Heilung offener Wunden oder und kutaner Läsionen bei Säugern jeglicher Art, einschließlich Verbrennungen, Photoalterung, Strahlenverletzungen sowie akuter und chronischer Wunden, und die Vorbeugung der Bildung von Läsionen in intakter Haut bei Säugern.

4. Antioxidanszusammensetzung zur Verwendung nach einem der Ansprüche 2 oder 3, wobei die Behandlung die therapeutische und/oder heilende Behandlung von akuten Traumata und chirurgischen Wunden, Schnittwunden, chirurgischen Dehiszenzen, Abschürfungen, Risswunden, Feuchtigkeitsläsionen, Platzwunden, Verbrennungen, Verbrühungen, Hautreizungen, Fisteln, venösen Ulzera, arteriellen Ulzera, Druckgeschwüren, diabetischen Fußulzera, Ulzera gemischter Ätiologie, entzündlichen Hautläsionen, Hautkrebs und chronischen oder komplexen Wunden einschließt.

5. Antioxidanszusammensetzung zur Verwendung nach einem der Ansprüche 2 bis 4, wobei die Verwendung eine topische Verwendung ist, die aus der Gruppe ausgewählt wird, die Folgendes umfasst: Creme, Lotion, Mikroemulsionsgel, Hydrogel, Salbe, Liposomen, Puder und wässrige Lösungen oder Suspensionen.

6. Antioxidanszusammensetzung zur Verwendung nach Anspruch 5 in Form eines Hydrogels, das eine Viskosität von 25000 bis 70000 mPa·s (RV 05 5 U/min) aufweist.

## Revendications

1. Composition antioxydante comprenant, par rapport au poids total de la composition, de 0,1 à 5 % de gomme de caroube, de 0,0001 à 1 % de lycopène et de 0,0001 à 1 % de curcuma.

2. Composition antioxydante selon la revendication 1, destinée à être utilisée dans le traitement thérapeutique de lésions cutanées chez les mammifères.

3. Composition antioxydante destinée à être utilisée selon la revendication 2, dans laquelle le traitement thérapeutique de la peau comprend l'amélioration du processus de cicatrisation et de guérison des plaies se produisant en présence d'espèces réactives de l'oxygène, incluant la cicatrisation de plaies ouvertes ou de lésions cutanées chez les mammifères, de tout type, incluant les brûlures, le photovieillissement, les lésions produites par un rayonnement et les plaies aiguës et chroniques, et à la prévention de la formation de lésions sur la peau intacte des mammifères.

4. Composition antioxydante destinée à être utilisée selon l'une quelconque des revendications 2 ou 3, dans laquelle le traitement inclut un traitement thérapeutique et/ou cicatrisant destiné à un traumatisme aigu et aux plaies chirurgicales, aux coupures, aux déhiscences chirurgicales, aux abrasions, aux lacérations, aux lésions dues à l'humidité, aux blessures, aux brûlures, aux échaudures, aux irritation cutanées, aux fistules, aux ulcères veineux, aux ulcères artériels, aux plaies de pression, aux maux perforants plantaires, aux ulcères d'étiologie mixte, aux lésions cutanées inflammatoires, au cancer de la peau et aux plaies chroniques ou complexes.

5. Composition antioxydante destinée à être utilisée selon l'une quelconque des revendications 2 à 4, dans laquelle l'utilisation est une utilisation topique choisie dans le groupe comprenant : une crème, une lotion, un gel du type microémulsion, un hydrogel, une pommade, des liposomes, des poudres et des suspensions ou des solutions aqueuses.

6. Composition antioxydante destinée à être utilisée selon la revendication 5 sous la forme d'un hydrogel présentant une viscosité allant de 25 000 à 70 000 mPa·s (RV 05 5 tr/min).
